# EUROPEAN PATENT SPECIFICATION

(11) **EP 0 571 538 B1**
(45) Date of publication and mention of the grant of the patent: **08.07.1998**
(21) Application number: 92907062.1
(22) Date of filing: 12.02.1992
(51) Int. Cl.: C12N 15/31, C12N 15/62, C12N 15/74, C12N 15/70, C07K 14/315, C12N 1/21, A61K 39/09, A61K 35/74, G01N 33/544

(54) **STRUCTURAL GENE OF PNEUMOCOCCAL PROTEIN**
STRUKTURGEN VON PNEUMOKOKKEN-PROTEIN
GENE DE STRUCTURE DE PROTEINE DE PNEUMOCOQUE

(30) Priority: 15.02.1991 US 656773
(43) Date of publication of application: 01.12.1993
(60) Divisional application: 96119668.0
(73) Proprietor: THE UAB RESEARCH FOUNDATION, Birmingham Alabama 35294 (US)
(72) Inventor: BRILES, David, E., Birmingham, AL 35222 (US); YOTHER, Janet, L., Birmingham, AL 35242 (US); McDANIEL, Larry, S., Birmingham, AL 35210 (US)
(74) Representative: Smart, Peter John
(86) International application number: PCT/US92/00857
(87) International publication number: WO 92/14488

(56) References cited:
- ABSTRACTS OF THE ANNUAL MEETING OF THE AMERICAN SOCIETY FOR MICROBIOLOGY May 1990 , WASHINGTON US page 98 J. YOTHER ET AL. 'Generation of truncated forms of pnaumococcal surface protein A (PspA) and DNA sequencing of pspA'
- INFECTION AND IMMUNITY vol. 59, no. 1 , January 1991 , WASHINGTON US pages 222 - 228 LARRY S. MCDANIEL ET AL. 'PspA, a surface protein of Streptococcus pneumoniae, is capable of eliciting protection against Pneumococci of more than one capsular type'
- JOURNAL OF BACTERIOLOGY vol. 174, no. 2 , January 1992 pages 610 - 618 JANET YOTHER ET AL. 'Truncated forms of PspA that are secreted from Streptococcus pneumoniae and their use in functional studies and cloning of the PspA gene'
- INFECTION AND IMMUNITY vol. 59, no. 4 , April 1991 , WASHINGTON US pages 1285 - 1289 DEBORAH T. TALKINGTON ET AL. 'A 43-kilodalton pneumococcal surface protein, PspA: Isolation, protective abilities, and structural analysis of the amino-terminal sequence'
- ABSTRACTS OF THE ANNUAL MEETING OF THE AMERICAN SOCIETY FOR MICROBIOLOGY May 1991 , WASHINGTON US page 97 JANET YOTHER ET AL. 'Cloning and expression of the gene encoding pneumococcal surface protein A (PspA) from Streptococcus pneumoniae into Escherichia coli'
- ABSTRACTS OF THE ANNUAL MEETING OF THE AMERICAN SOCIETY FOR MICROBIOLOGY May 1991 , WASHINGTON US page 31 LARRY S. MCDANIEL ET AL. 'Localization of protective epitopes on PspA (pneumococcal surface protein A)'
- Abstracts of the 89th Annual Meeting of the ASM, 14-18 May 1989, McDANIEL et al., "Molecular Cloning of the Gene Encoding PspA (Pneumococcal Surface Protein A) and Analysis of Protective Immunity Induced by PspA", see Abstract No. D-255.
- Microbial Pathogenesis, Vol. 1, issued 1986, McDANIEL et al., "Analysis of a Surface Protein of Streptococcus pneumoniae recognized by Protective Monoclonal Antibodies", pages 519-531, see entire document.
- Journal of Experimental Medicine, Vol. 165, issued February 1987, McDANIEL et al., "Use of Insertional Inactivation to Facilitate Studies of Biological Properties of Pneumococcal Surface Protein A (PspA)", pages 381-394, see entire document.

## Description

The present invention is concerned with the development of an improved vaccine against pneumococcal infections.

### BACKGROUND TO THE INVENTION

Streptococcus Pneumoniae is an important cause of otitis media, meningitis, bacteremia and pneumonia. Despite the use of antibodies and vaccines, the prevalence of pneumococcal infections has declined little over the last twenty-five years.

It is generally accepted that immunity to Streptococcus pneumoniae can be mediated by specific antibodies against the polysaccharide capsule of the pneumococcus. However, neonates and young children fail to make an immune response against polysaccharide antigens and can have repeated infections involving the same capsular serotype.

One approach to immunising infants against a number of encapsulated bacteria is to conjugate the capsular polysaccharide antigens to protein to make them immunogenic. This approach has been successful, for example, with Haemophilus influenzae b (see U.S. Patent No. 4,496,538 to Gordon and U.S. Patent No. 4,673,574 to Anderson). However, there are over eighty known capsular serotypes of S. pneumoniae of which twenty-three account for most of the disease. For a pneumococcal polysaccharide-protein conjugate to be successful, the capsular types responsible for most pneumococcal infections would have to be made adequately immunogenic. This approach may be difficult, because the twenty-three polysaccharides included in the presently-available vaccine are not all adequately immunogenic, even in adults.

An alternative approach for protecting children, and also the elderly, from pneumococcal infection would be to identify protein antigens that could elicit protective immune responses. Such proteins may serve as a vaccine by themselves, may be used in conjunction with successful polysaccharide-protein conjugates, or as carriers for polysaccharides.

In McDaniel et al (I), J.Exp.Med. 160:386-397, 1984, there is described the production of hybridoma antibodies that recognise cell surface polypeptide(s) on S. pneumoniae and protection of mice from infection with certain strains of encapsulated pneumococci by such antibodies. This surface protein antigen has been termed "pneumococcal surface protein A" or PspA for short.

In McDaniel et al (II), Microbial Pathogenesis 1:519-513, 1986, there are described studies on the characterisation of the PspA. Considerable diversity in the PspA molecule in different strains was found, as were differences in the epitopes recognised by different antibodies.

In Yother et al, Abstracts of the 90th Annual Meeting of the American Society for Microbiology, 13-17 May 1990, page 98 Abstract No. D-106, there is disclosed the production of truncated PspA proteins having from 40 to 90% of the full length PspA molecular weight which are released from the cell expressing them. The abstract however lacks details of the production of these materials.

In McDaniel et al (III), J.Exp.Med. 165:381-394, 1987, there is disclosed that immunisation of X-linked immunodeficient (XID) mice with non-encapsulated pneumococci expressing PspA, but not isogenic pneumococci lacking PspA, protects mice from subsequent fatal infection with pneumococci.

In McDaniel et al (IV), Infect. Immun., 59:222-228, 1991, there is described immunisation of mice with a recombinant full length fragment of PspA that is able to elicit protection against pneumococcal strains of capsular types 6A and 3.

In Crain et al, Infect.Immun., 56:3293-3299, 1990, there is described a rabbit antiserum that detects PspA in 100% (n = 95) of clinical and laboratory isolates of strains of S. pneumoniae. When reacted with seven monoclonal antibodies to PspA, fifty-seven S. pneumoniae isolates exhibited thirty-one different patterns of reactivity.

The PspA protein type is independent of capsular type. It would seem that genetic mutation or exchange in the environment has allowed for the development of a large pool of strains which are highly diverse with respect to capsule, PspA, and possibly other molecules with variable structures. Variability of PspA's from different strains also is evident in their molecular weights, which range from 67 to 99 kD. The observed differences are stably inherited and are not the result of protein degradation.

Immunization with a partially purified PspA from a recombinant λ gt11 clone, elicited protection against challenge with several S. pneumoniae strains representing different capsular and PspA types, as described in McDaniel et al (IV), Infect. Immun. 59:222-228, 1991. Although clones expressing PspA were constructed according to that paper, the product was insoluble and isolation from cell fragments following lysis was not possible.

While the protein is variable in structure between different pneumococcal strains, numerous cross-reactions exist between all PspA's, suggesting that sufficient common epitopes may be present to allow a single PspA or at least a small number of PspA's to elicit protection against a large number of S. pneumoniae strains.

In addition to the published literature specifically referred to above, the inventors, in conjunction with co-workers, have published further details concerning PspA's, as follows:
1. Abstracts of 89th Annual Meeting of the American Society for Microbiology, p. 125, item D-257, May 1989;
2. Abstracts of 3rd International ASM Conference on Streptococcal Genetics, p. 11, item 12, June 1990;
3. Talkington et al, Infect. Immun. 59:1285-1289, 1991;
4. Yother et al, J. Bacteriol. 174:601-609, 1992; and
5. Yother et al, J. Bacteriol. 174:610-618, 1992.

The latter three publications occurred after the filing of the priority basis of this application.

In the specification which follows and the drawings accompanying the same, there are utilised certain accepted abbreviations with respect to the amino acids represented thereby. The following Table I identifies those abbreviations.

**TABLE I**

| **AMINO ACID ABBREVIATIONS** | |
|---|---|
| A = Ala = Alanine | M = Met = Methionine |
| C = Cys = Cysteine | N = Asn = Asparagine |
| D = Asp = Aspartic Acid | P = Pro = Proline |
| E = Glu = Glutamic Acid | Q = Gln = Glutamine |
| F = Phe = Phenylalanine | R = Arg = Arginine |
| G = Gly = Glycine | S = Ser = Serine |
| H = His = Histidine | T = Thr = Threonine |
| I = Ile = Isoleucine | V = Val = Valine |
| K = Lys = Lysine | W = Try = Tryptophan |
| L = Leu = Leucine | Y = Try = Tyrosine |

### SUMMARY OF INVENTION

The present invention relates to the preparation of mutants of S. pneumoniae that secrete an immunogenic truncated form of the PspA protein, and isolation and purification of the secreted protein. The truncated form of the PspA protein is immunoprotective and contains at least one protective epitope of PspA. The PspA protein is soluble in physiologic solution and lacking at least the functional cell membrane anchor region.

### BRIEF DESCRIPTION OF DRAWINGS

Figure 1 is a schematic representation of the domains of the mature PspA;
Figure 2 is the N-terminal amino acid sequence of PspA, wherein upper case letters denote charged hydrophilic amino acids;
Figure 3 is the DNA sequence of the pspA gene with deduced amino acid sequence for the PspA protein;
Figure 4 depicts the restriction map of pspA (Figure 4A) and the use of insertion-duplication mutagenesis to construct mutations in the pspA gene (Figure 4B);
Figure 5 shows the deduced amino acid sequence for the N-terminal region of PspA and the general location of epitopes recognised by monoclonal antibodies;
Figure 6 shows antibody reactivity with PspA fragments produced by various pspA gene segments; and
Figure 7 shows the mapped location of epitopes in the PspA fragments produced by the different pspA gene segments.

### GENERAL DESCRIPTION OF INVENTION

According to one aspect of the present invention, there is provided a purified immunoprotective pneumococcal surface protein, comprising a truncated form of PspA which contains at least one immunoprotective epitope of the protein and up to about 90% of the whole PspA protein and from which the functional cell membrane anchor region is absent.

Through the technique of insertion-duplication mutagenesis of the pspA gene of the strain Rx1 of Streptococcus pneumoniae with plasmids containing cloned fragments of the pspA structural gene, it has been possible to produce soluble fragments of PspA that are secreted by pneumococci.

In another aspect of the present invention, therefore, there is provided a method for forming an immunoprotective truncated PspA protein, characterised by:
(a) effecting insertion-duplication mutagenesis of a bacterium with a pspA gene resulting in the coding of a truncated expressible pneumococcal surface protein (PspA) from which the functional cell membrane anchor region is absent, or
(b) fusing a pspA gene coding for a truncated form of PspA protein from which the functional cell membrane anchor region is absent with a gene coding for another protein to form a fusion protein clone and transforming a bacterium with said fusion protein clone,
   growing said bacterium to effect expression of a truncated PspA protein or a fusion protein comprising truncated PspA and said other protein, and
   isolating the expressed protein.

The molecular size of the purified truncated PspA protein obtained may be varied by directing the point of insertion, which determines the termination of gene expression, to different points in the pspA gene. For example, an N-terminal fragment of apparent molecular weight of 43 kD, constituting approximately one-half of the native protein, has been found useful.

The truncated segment which is produced by this procedure is capable of eliciting protection in mice from fatal challenge with type 3 S. pneumoniae, demonstrating for the first time that a purified PspA can elicit protection and that this truncated segment of the protein contains protective epitopes of PspA.

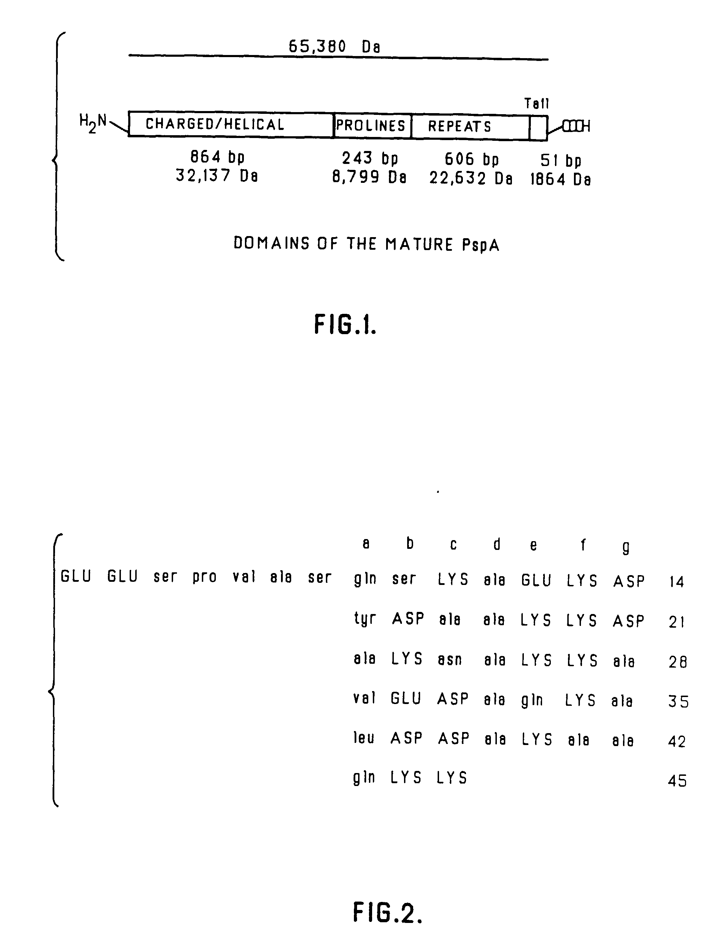

Amino acid sequence information was obtained on the N-terminal 45 amino acids of the truncated segment of PspA. This sequence is shown in Figure 2. Predictive secondary structural analysis shows that this sequence has a very strong alpha-helical formation, with no non-helical inserts. About 51% of the segment is composed only of two amino acids, namely lysine, a charged amino acid, and alanine, a non-polar amino acid.

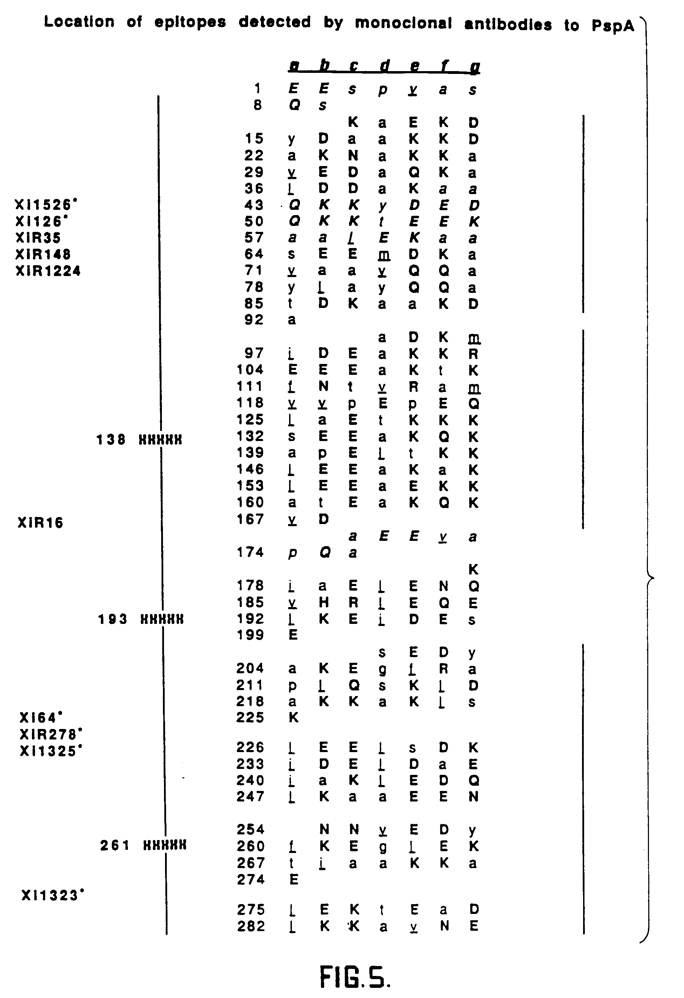

Analysis of this 45-amino acid sequence also reveals that it contains a seven-residue periodicity (see Figure 2). In PspA, the periodicity begins with residue 8 and extends throughout the entire sequence, for nearly eleven turns of the helix. Positions "a" and "d" are occupied by apolar amino acids and position "b", "c" and "f" generally contain hydrophilic amino acids. Position "f" is predominantly occupied by lysine. Having regard to these observations, this region of PspA is very likely in an alpha-helical coiled-coil configuration. The deduced amino acid sequence for the whole of the α-helical coiled-coil region is shown in Figure 5.

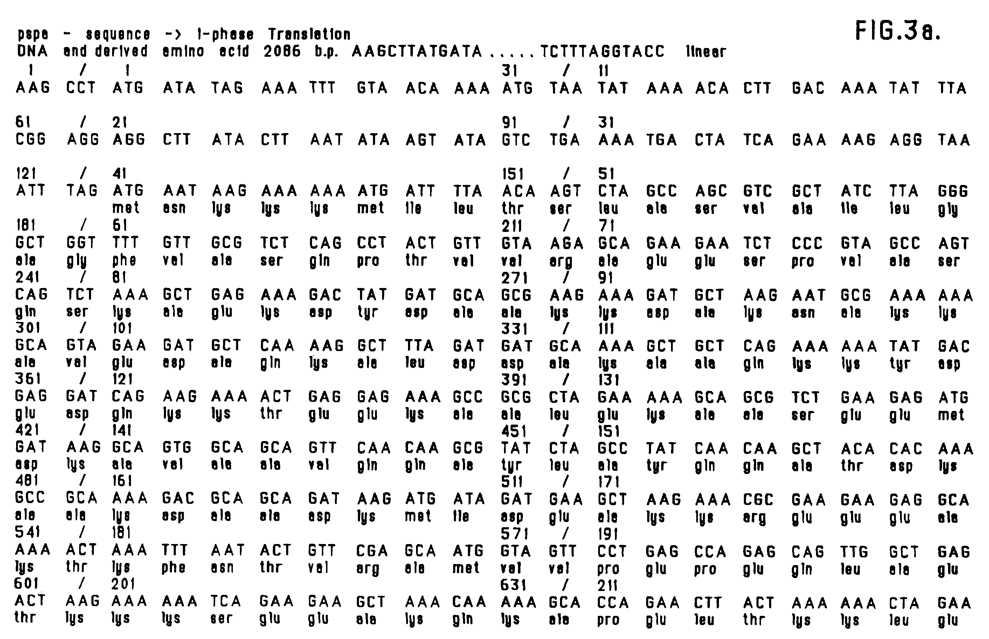

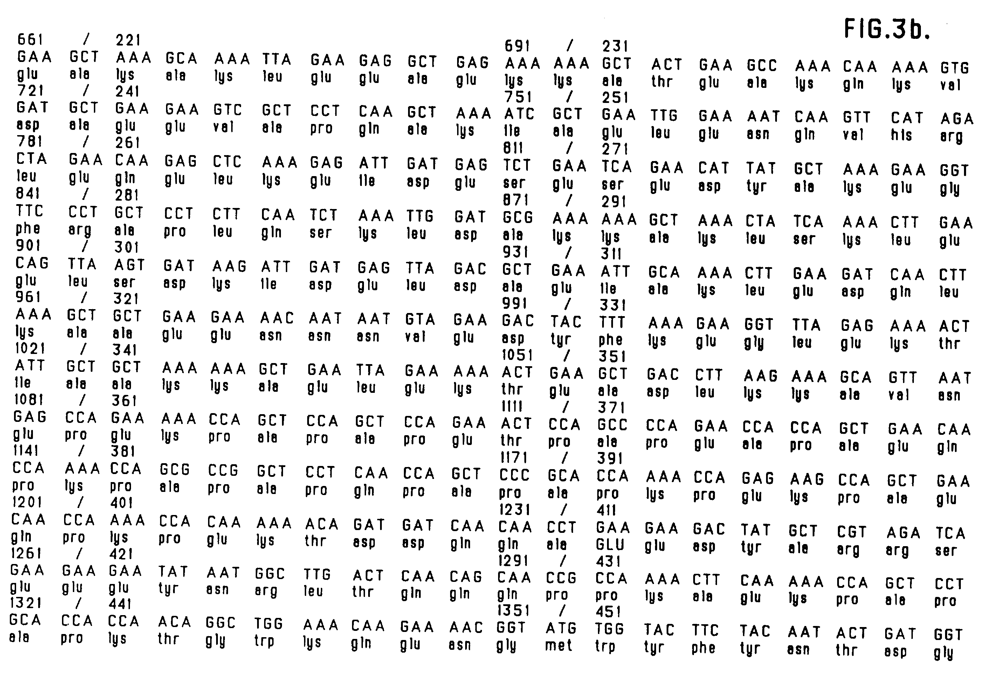

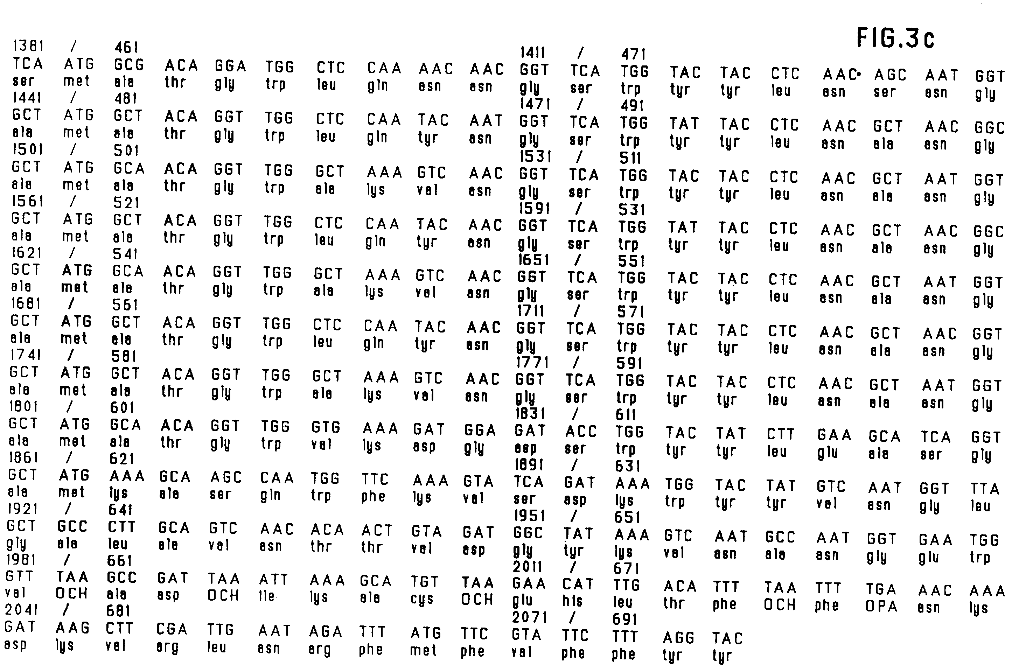

We also have cloned and sequenced the entire coding region of pspA (see Figure 3). The deduced amino acid sequence for the PspA protein reveals three distinct regions of the PspA molecule, shown schematically in Figure 1. Accordingly, a further aspect of the present invention, there is provided a biologically-pure recombinant DNA molecule coding for said truncated form of the PspA protein and having a coding sequence included within that set forth in Figure 3.

The DNA sequence of the pspA gene is contained on a HindIII - KpnI fragment that is 2086 base pairs in length. The pspA gene itself represents approximately 1985 base pairs of this fragment, and comprises an initial region containing transcription and translational signals with translation starting at the ATG/met (nucleotide position 127, codon position -31), followed by a leader sequence extending from the ATG/met (nucleotide position 127, codon position -31) to CGA/ala (nucleotide position 217, codon -1). Mature Pspa starts with the Glu amino acid at nucleotide position 220 (codon +1) and ends at the translational stop TAA/OCH at nucleotide position 1984. This translational stop codon is followed by transcription termination signals.

The amino terminal of the protein sequence, predicted from the DNA sequence of Figure 3, contains a 31 amino acid leader sequence and a 45 amino acid sequence identical to the 45 amino acid sequence of the N-terminal of PspA (Figure 2). The amino end of the predicted protein sequence is highly charged and α-helical in nature. This region has homology with tropomyosin at the amino acid level (approximately 22% identity and 50% similarity). This homology is due largely to a repeating seven residue periodicity where the first and fourth amino acids are hydrophobic, the intervening amino acids are helix-promoting and the seventh amino acid is charged. This pattern is consistent with that of an α-helical coiled-coil molecule and indicates that the α-helical coil extends through the N-terminal half of the molecule. The amino acid sequence of the whole of the α-helical coil region is shown in Figure 5.

Following the charged helical region is a proline-rich region in which 23 of 81 amino acids are prolines. Immediately carboxy to the proline-rich region is the first of ten highly homologous twenty amino acid repeats. The only significantly hydrophobic region in the sequenced portion of the molecule begins at the last repeat. This potential membrane-spanning region contains several charged amino acids preceding the translational stop codon.

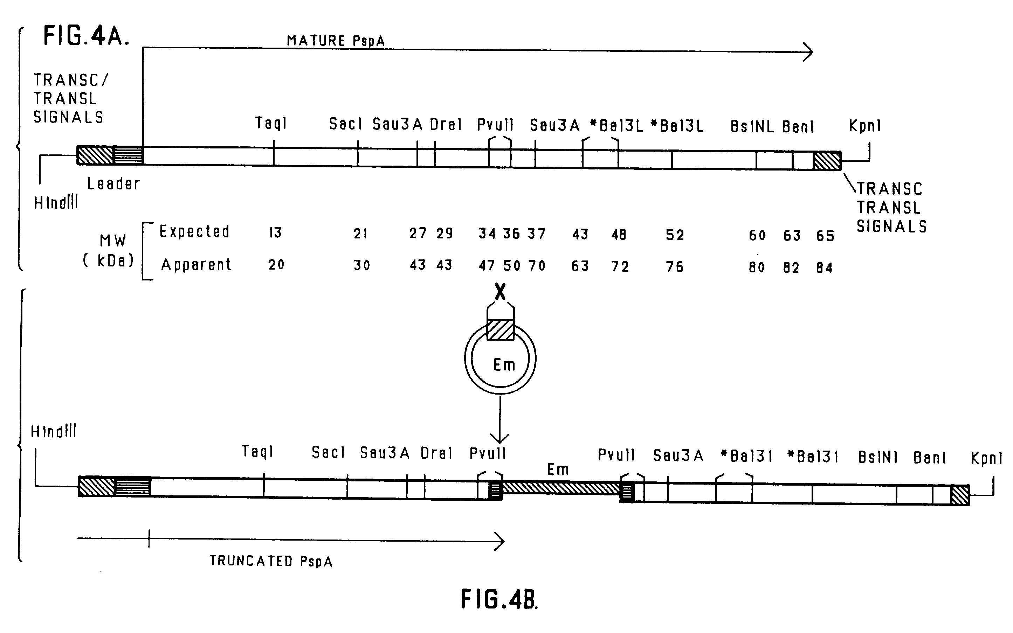

The insertionally-inactivated mutants of S.pneumoniae lacking the C-terminal anchor regions are capable of growth in chemically-defined medium and secrete the N-terminal portion of the PspA protein into the medium. The N-terminal region of PspA is highly soluble in the culture medium and is much easier to isolate than the entire molecule. Soluble truncated molecules have been produced using insertional duplicational mutagenesis directed by the cloned PspA DNA fragments shown in Figure 4. Expression of the same truncated construct (with the pneumococcal promoter) in E.coli results in the same PspA fragment being secreted into the periplasm of E.coli. PspA is readily released from the periplasm by hypotonic lysis.

Truncated PspA is isolated from culture medium of mutant pneumococci in any convenient manner, such as by tangential flow filtration. Ion-exchange chromatography then is performed on an anionic resin to purify the protein. In this procedure, the solution containing PspA is dialyzed to pH6 in 0.02 M salt solution and passed over the resin. The PspA is eluted from the resin with a gradient of 0.08 to 2.0 M ionic strength and is collected in the fraction between 0.34 and 0.87 M ionic strength, depending on the nature of the column used.

The PspA may be further purified by sodium dodecyl sulfate polyacrylamide gel (SDS-PAGE) electrophoresis. The PspA-containing portion of the gel is identified by staining of the gel and PspA is electroeluted from this portion.

The electrophoresis purification is convenient when only small quantities of PspA are being handled. As an alternative, more suited to large-scale production, the protein may be purified by size chromatography in a pH7 phosphate buffer.

Since it is possible to obtain expression of the truncated form of PspA into the culture medium, as opposed to it being trapped within the cell wall and making purification much more complicated, it is possible to isolate other proteins that have been cloned into the truncated pspA gene by making fusion proteins between PspA and other proteins. Such a technique may be employed to enhance the immunogenicity or preserve the immunogenic structural conformation or presentation of the gene product, to permit the fusion protein to be used in immunization, which may be systemic and/or mucosal, against disease.

One example of such a fusion protein is a fusion of the soluble N-terminal region of PspA and the B-subunit of cholera toxin. Fusion proteins also may be formed by chemical attachment of the truncated PspA protein to other proteins.

Another aspect of the present invention, therefore, provides a method for the production of cloned proteins, which comprises fusing a pspA gene coding for a truncated form of PspA protein with the gene coding for another protein to form a fusion protein clone, transforming S.pneumoniae, E.coli or other bacteria with the fusion protein clone, growing the transformed bacterium to effect expression of a fusion protein comprising truncated PspA and the other protein into the culture medium, and isolating the fusion protein.

By using this technique, there can be produced cloned proteins in gram positive bacteria, such as pneumococci, for example, S.pneumoniae, and mycobacteria, for example, Bacille Calmette-Guerin (BCG). This approach overcomes the problems inherent in the production of proteins in gram negative bacteria, such as E. coli, usually used for cloning, in particular the need to purify the recombinant proteins from endotoxin and the toxicity of many gram positive DNA sequences in gram negative organisms.

For the expression of a fusion protein comprising the soluble N-terminal region of PspA and the B-subunit of cholera toxin (CTB), a gene fusion of a pspA gene coding for a truncated form of PspA protein with a ctxB gene coding for the B-subunit of cholera toxin is effected. Following expression of the fusion protein, the PspA and CTB may be cleaved one from another by dilute acid at an asparagine-proline sequence, known to be labile to dilute acid, engineered at the fusion site of the two proteins.

CTB is known to be highly specific for monosinloganglioside (G_{M1}). Accordingly, the fusion PspA-CTB protein may be isolated from the culture medium by adsorption to a G_{M1} affinity column, from which the fusion protein subsequently may be eluted at low pH.

The PspA-CTB fusion protein finds considerable utility in solid phase immunoadsorbant assays. By using the fusion protein, it is possible to coat solid supports, such as microtitration plates, with PspA fragments without having first to isolate the PspA fragments. This may be done by adding bacterial extract containing the fusion protein to plates coated with G_{M1}. The PspA-CTB fusion protein then binds to G_{M1} through the CTB moiety, thereby coating the solid support with PspA. The resulting coated product then may be used in a solid phase immunoadsorbant assay for the detection of PspA antibody and/or antigen in test samples. Such immunoadsorbant assays constitute an additional aspect of this invention.

The PspA attachment/anchor region, containing the proline-rich region, the repeat region and/or the C-terminus of PspA, also may be employed to effect expression of heterologous proteins in pneumococci, or other gram positive or gram negative bacteria in the which the attachment/anchor region is functional. Generally, expression is effected on bacterial membrane, cell walls or cell surfaces in gram positive bacteria and in the periplasm of gram negative bacteria. An example of such heterologous protein is the B-subunit of cholera toxin.

As mentioned above, the truncated form of PspA provided herein contains the immunoprotective epitopes of the protein and hence is useful in a vaccine against pneumococcal infection. Accordingly, a yet further aspect of the present invention provides a vaccine against pneumococcal infection comprising, as an immunogenically-active component, the purified immunoprotective pneumococcal surface protein provided herein. The PspA protein may be employed as one component of a multicomponent vaccine which is effective in providing protection from a variety of infections.

In addition, gram positive bacteria which have been transformed to express the pspA gene coding for the truncated soluble PspA protein may be employed, in a live-attenuated or killed form, as an immunologically-active component of a vaccine against pneumococcal infection. In the transformed bacterium, such pspA gene may be fused to a gene coding for another protein. Accordingly, an additional aspect of this invention provides a vaccine against pneumococcal infection comprising, as an immunologically-active component, a live-attenuated or killed bacteria containing a gene coding for the truncated form of PspA.

The truncated form of PspA also may be employed in conjugates with normally weakly-immunogenic or non-immunogenic protection-eliciting molecules, such as various polysaccharides, to achieve immunogenic potentiation thereof. An additional aspect of the invention, therefore, provides a vaccine comprising, as an immunogenically-active component, a conjugate of the purified immunoprotective pneumococcal surface protein provided herein and a normally weakly-immunogenic or non-immunogenic protection-eliciting molecule.

Conserved sequences of pspA, particularly those in the proline-rich and/or repeat regions of the gene, may be employed as probes to detect the presence of pneumococci of various strains, through detection of pneumococcal DNA, in tissues, body fluids and/or secretions. Similarly, portions of the pspA gene may be used in diagnostic kits for the detection of pneumococcal infections.

In addition, primers made based on conserved sequences of pspA, particularly those in the proline-rich and/or repeat regions, may be used to assay for the presence of pneumococci in tissues, body fluids and/or secretions, through amplification of pneumococcal DNA. In this regard, a single primer pair derived from the nucleotide sequence of the pspA gene of S.pneumoniae may be employed in an assay using the polymerase chain reaction (PCR) for the specific detection of Streptococcus pneumoniae.

Specific amplification has been achieved of a 678 base pair DNA fragment from S.pneumoniae strain Rx1. After 30 cycles of amplification, the amplimer was detectable by agarose gel electrophoresis. The fragment was successfully amplified in all 32 strains of S.pneumoniae tested. PCR DNA amplification was able to detect less than an estimated 20 picograms total genomic pneumococcal DNA.

Primers LSM1 and LSM2, having the nucleotide sequences: 
LSM1 5'-CCGGATCCAGCTCCTGCACCAAAAC-3'
LSM2 5'-GCGCTGCGACGGCTTAAACCCATTCACCATTGG-3'
 amplified the 678 base pair product from pspA from nucleotides 1312 to 1990 of the Rx1 pspA sequence (Figure 3).

The PCR analysis using the primers described herein is performed in accordance with conventional PCR techniques, such as are described in the literature, for example, as described in Arnhem et al at C&EN Special Report, 36, October 1, 1990. For detection purposes, the primer may be labelled or labelled nucleotide triphosphates may be included in the PCR reaction to label the PCR amplification product.

The PCR primers may be prepared by well-known methods, for example, by oligonucleotide synthesis or by fragmentation of a larger nucleotide sequence using suitable restriction enzymes.

The ability to use a single primer capable of detecting a large number of S.pneumoniae strains enables a universal PCR detection kit to be provided which is able to diagnose pneumococcal infection in mammals, including humans, independent of the strain which has caused the disease.

### STRAINS, PLASMIDS AND PROBES

In the Examples which follow as well as in the accompanying drawings, reference is made to certain plasmids and bacterial strains transformed by such plasmids as well as vector DNA segments, some of which have been deposited with ATCC and all of which are fully described herein. The following Table II provides a summary of such materials.

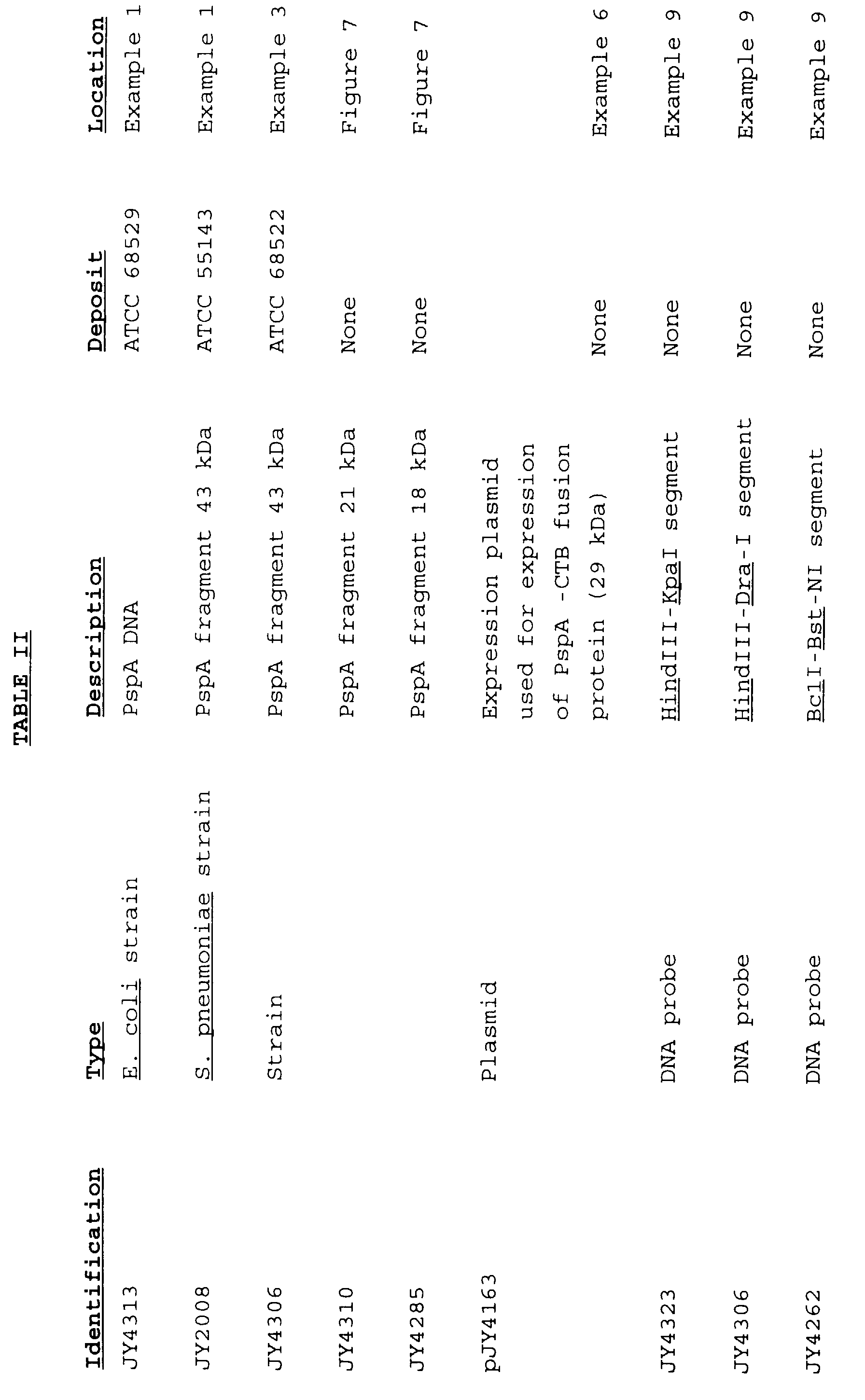

### EXAMPLES

### Example 1

This Example illustrates the preparation and growth of novel strains of S. pneumoniae.

The S. pneumoniae strain Rx1, which is a non-encapsulated derivative of capsular type 2 strain D39 (National Collection of Type Cultures, London, NCTC #7466), was subjected to insertional inactivation (as described in McDaniel et al (III) 1987, Crain et al 1990, Talkington et al 1991, with 10 different cloned fragments of PspA (see Figure 4). These fragments have all been obtained from restriction digests of cloned PspA DNA on a plasmid in E.coli strain JY4313 (deposited with the American Type Culture Collection on January 31, 1991 under ATCC accession number 68529). This insertional duplication mutagenesis (see Figure 4) results in the termination of gene expression near the 3' end of the cloned fragment.

One of the resultant strains, JY2008 (deposited with the American Type Culture Collection on January 24, 1991 under accession number 55143), which was produced by a fragment of DNA encoded in pKSD300 (McDaniel et al (III) 1987), produces a PspA fragment of 27 kDa (apparent molecular weight 43 kDa). This fragment is approximately 40% the size of the native 65 kDa (84 kDa apparent size) protein.

The expected molecular size is based on the deduced amino acid sequence and the apparent molecular size is based on migration in SDS-PAGE. The difference between expected and apparent molecular size is due to the conformation of the PspA fragment.

The proline and repeats/anchor regions (see Figure 1) were deleted and the resulting protein was unable to attach to cell due to their absence. The unattached protein then may be isolated from culture supernatants, as described below.

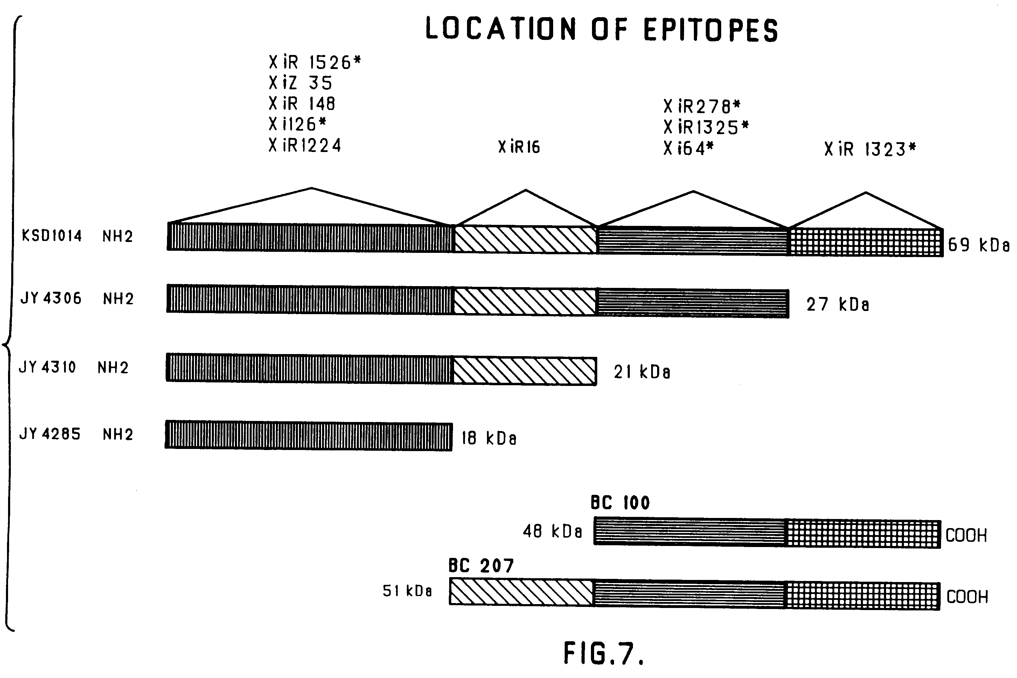

By directing the insertion to different points in the pspA gene, different lengths of truncated, non-attached PspA protein derivatives can be produced, as seen in Figure 7.
The pneumococcal strain JY2008 was grown in 6 liters of a chemically defined medium (see Inf. Imm. 27:444) supplemented with 0.10% choline chloride, 0.075% L-cysteine hydrochloride and 0.25% NaHCO₃. The supernatant fluid of the mid-log phase culture of JY2008 was harvested using a 0.22 µm membrane tangential flow filter and concentrated 60 fold.

Introduction of the plasmid pKSD300 into the unmodified D39 strain similarly yielded the 43 kD truncated PspA protein. Introduction of the plasmid pKSD300 into the type 3 S.pneumoniae strain WU2 (PspA protein approximately 92 kD) yielded, upon growth of the organism, a non-attached truncated PspA protein of approximately 46 kD molecule size.

### Example 2

This Example illustrates the purification of PspA.

The concentrated supernatant fluid, produced as described in Example 1, was washed in 0.1 M PBS, pH 7.2, and ultracentrifuged at 196,000 xg. The supernatant fluid was diluted 1:5 in 20 mM L-histidine buffer-NaCl, the pH adjusted to 6.0 and the injected into a DEAE-fibered Isonet-D2^{TM} an ion exchange column.

A stepwise NaCl gradient from 80 mM to 2 M was applied to the column and PspA-containing fractions (0.32 to 0.64 M ionic strength) were pooled and separated on an SDA-polyacrylamide gel. The proteins on a representative section of the gel were stained with Comassie Blue R-250 to identify PspA. The fraction containing PspA was excised from the remainder of the SDS-gel and electroeluted from the excised gel. The eluted protein was precipitated in a 50:50 methanol:acetone solvent and resuspended in PBS. Purity of product was confirmed by silver staining and Western Immunoblotting with mAb Xi126 (IgG 2b, k, see McDaniel et al (I), supra).

### Example 3

This Example illustrates the isolation of PspA from the bacterial periplasmic space.

Isolation from the periplasmic space was accomplished by standard techniques. Strain JY4306 (which produces the 43 kDa N-terminal fragment of PspA, the amino acid sequence of which is shown in Figure 3. This strain was deposited with ATCC on January 31, 1991 under accession number 68522, was washed in buffered saline, incubated in 20% sucrose, 10mM EDTA, 25mM Tris pH 7.7 for 10 minutes at 0°C. The cells then were spun at 400 xg for 10 minutes at 0°C. All supernatant was removed from the pellet and the pellet was resuspended rapidly in about 100 volumes of 4°C water. After 10 minutes the suspension was centrifuged at 4,000 xg for 10 minutes at 4°C. The pellet was discarded and the supernatant, which contained the PspA was saved. Concentration of the supernatant was by standard procedures such as concentration against solid sucrose or ultrafiltration. Purification of the protein isolated from E. coli proceeded by the same chromatography techniques used for the isolation of the 43 kDa (truncated) PspA from the media of growing pneumococci.

### Example 4

This Example illustrates the immunogenic properties of the PspA protein.

Sixteen 7-week old CBA/N mice carrying the Xid mutation (Jackson Laboratories, Bar Harber, ME) were bled via the periorbital sinus to establish pre-exposure levels of antibody to PspA. Purified PspA, prepared as described in Example 2, was emulsified in complete Freund's adjuvant and injected subcutaneously into the inguinal and auxiliary regions, delivering approximately 5 µg of protein per mouse. Fourteen days later, the mice were injected intraperitoneally with 5 µg of PspA prepared as described in Example 2. Control mice were immunized via the same routes with sterile SDS buffer. Seven days after the last immunization, all mice were bled via the periorbital sinus and were challenged intravenously with 300 CFU of the type 3 strain WU2, grown as described in Example 1.

Preimmunization and prechallenge sera were analyzed by Western immunoblots to establish baseline and postimmunization response to the truncated protein. The PspA of strain WU2 was electrophoresed and transferred to nitrocellulose membranes. The membranes were separated into strips and probed with the appropriate mouse antisera at a 1:50 dilution for 2 hours, incubated with biotinylated goat anti-mouse immunoglobulin for 1 hr, washed and incubated with Strepavidin-conjugated phosphatase. The membranes were developed with 5-bromo-4-chloro-3-indoyl phosphate toludine salt with 0.01% into blue tetrazolium.

Of the eight CBA/N mice immunized with the purified fragment of PspA, all were still alive 14 days after challenge with strain WU2 and none showed any signs of illness following challenge. Of the eight mice immunized with buffer controls, six were dead by two days post challenge, while the two remaining control mice appeared very sick, with ruffled fur, arched back and decreased movement, two to three days following challenge but survived. Chi-square analysis indicated that there was a significant difference (P <0.003) in survival between the immunized and control groups.

Preimmunization and prechallenge sera were analyzed by Western immunoblotting. None of the preimmunization sera contained antibody to truncated PspA. Postimmunization sera from eight of eight mice contained detectable antibodies to PspA, and six mice had very strong anti-PspA reactions. When the challenge strain WU2 was probed with the antisera, all the immunized mice had antibodies that were highly cross-reactive with the WU2 PspA epitopes. No control mice developed antibodies to PspA.

The immunization data is summarized in the following Table III:

**Table III**

| Immunogen | Detection of Antibody to PspA | Alive at 2 days post challenge | Alive at 14 days post challenge |
|---|---|---|---|
| Isolated PspA (Example 2) | 8/8 | 8/8 | 8/8 |
| Sterile SDS (control) | 0/8 | 2/8 | 2/8 |

As may be seen from the data in Table III, immunization with two 5 µg doses of the purified PspA molecule elicited protection against fatal infection of CBA/N mice and elicited antibodies reactive with the PspA of the challenge strain.

### Example 5

This Example illustrates sequencing of the PspA protein.

Purified PspA, prepared as described in Example 2, was electrophoresed through 9% resolving gels containing recrystallized SDS with the Laemmli buffer system (Nature 227:680). The gels were soaked twice in a 10mM 3-(cyclohexylamino)-1-propanesulfonic acid, pH 11.0, containing 10% methanol for 10 minutes. A polyvinylidene difluoride membrane (PVDF) was wetted completely for several seconds in 100% methanol, then washed in CAPS buffer for 10 min. PspA was electrotransferred to the PVDF membrane in CAPS buffer at 0.5 A for 1 hr. After transfer, the membrane was washed two times in deionized water for 5 min, and stained with 0.1% Coomassie Blue R-250 in 50% methanol for 20 minutes. The section of the membrane containing PspA was excised and destained in 40% methanol and 10% acetic acid for 5 min. The membrane was cut into small segments and stored in sterile Eppendorf^{TM} tubes until sequencing.

The isolated PspA was sequenced directly from the PVDF membranes. Figure 2 depicts the N-terminal 45 residue amino acid sequence and Figure 5 depicts the amino acid sequence for the whole alpha-helical region. The DNA sequence of the whole pspA gene and the deduced amino acid sequence for the PspA protein are shown in Figure 3.

### Example 6

This Example illustrates the use of the pspA 5'-sequence and/or the PspA N-terminal region to serve as an expression and leader sequence for expressing and/or excreting/secreting heterologous proteins from S.pneumoniae and E.coli. In this Example, there is described the expression of the N-terminal of the PspA protein fused to the B-subunit of cholera toxin (CTB) through a genetic fusion and the excretion of the fused protein from pneumococci and its secretion into the periplasmic space of E.coli.

A fusion protein consisting of CTB and the N-terminal half of PspA was constructed and expressed in E.coli. The HindIII/Dral pspA gene fragment used contained all the pspA transcription and translation initiation signals and the PspA signal peptide leader sequence for transport across the cell membrane. The mature PspA encoded by this fragment is predicted to be a product of 29 kDa (observed molecular weight of 42 kDa), encompassing more than 90% of the α-helical coiled-coil domain. The CTB fragment used lacked transcription and translation initiation signals. Expression from pspA promoter through pspA and then in-frame translational readthrough into the CTB-encoding gene ctxB resulted in production of a 12 kDa CTB product fused to the upstream PspA product. The PspA-CTB fusion protein was stably expressed in both high and low copy number plasmids (pUC18, more than 100 copies/cell; pJY4163, about 15 to 30 copies/cell) in E.coli.

The fusion products were of the expected size (about 54 kDa) and reacted with antibody to both PspA and CTB. That the CTB product retained its functionality was demonstrated by the ability of the fusion protein to bind ganglioside G_{M1}, a property of CTB.

The high level of expression of the fusion product apparently resulted in a reduced rate of processing and/or conformational changes that prevented the protein from being completely transported to the periplasm. However, in the lower copy number construct, about 60% of the fusion protein was localized in the periplasm, where large quantities were readily released from E. coli by osmotic shock.

In addition to expression in E.coli, the fusion protein also was expressed in S.pneumoniae by transformation of the low copy number construct into the avirulent S.pneumoniae Rx1 to generate an insertion-duplication mutant. In this way, the gene encoding the fusion protein was integrated into the S.pneumoniae chromosome, from which it was stably expressed. As in the case of Example 1, the truncated PspA molecule lacking the attachment/anchor region, this time in the form of the PspA-CTB fusion protein, was excreted into the culture supernatant. The fusion protein product was of the expected molecular size (54 kDa), reacted with antibody to PspA and CTB, and bound G_{M1}.

### Example 7

This Example illustrates the use of PspA attachment or anchor region to permit expression of heterologous proteins on the surface of S.pneumoniae or other bacteria in which the attachment/anchor sequence is functional in particular the expression of a PspA-CTB (cholera toxin B subunit) fusion expressed on the surface of pneumococci.

The N-terminal encoding region of PspA, including its transcription and translation initiation signals and its signal peptide leader sequence, is linked via a translationally in-frame genetic fusion to the CTB-encoding ctxB fragment that lacks transcription and translation initiation and termination signals. This sequence is followed in-frame by the PspA attachment/anchor domain, including part or all of the proline, repeat and C-terminal domains. The resulting fusion protein is directed to the outside of the cell via the PspA leader sequence, which is cleaned following transport across the membrane, and then attached to cell by the PspA attachment/anchor sequence. The heterologous protein, located between the two PspA fragments is expressed on the outside surface of the membrane and, in S.pneumoniae, on the surface of the cell.

### Example 8

This Example illustrates the expression of truncated and full length PspA by the Mycobacterium tuberculosis strain Bacille Calmette-Guerin (BCG).

BCG was chromosomically modified to incorporate the pspA gene coding for the truncated PspA protein. The 43 kDa truncated PspA protein was expressed from the modified BCG to approximately 15% of total BCG protein. This result was achieved with an expression vector construct carrying the pspA gene segment encoding the 43 kDa region without its 5'-secretion signal. Expression was only about 1% of BCG protein when the PspA or mycobacterial signal sequences were included. In either case, a significant portion of the expressed PspA was excreted into the medium. Expression of the 43 kDa PspA protein in a fusion with the mycobacterial lipoprotein signal sequence resulted in the expression of the recombinant PspA in the membrane fraction of BCG.

This latter result suggested that the fusion of the lipoprotein signal sequence resulted in acylation of the recombinant PspA. Fluorescent activated cell sorting with fluorochrome-conjugated monoclonal antibodies to PspA demonstrated expression of PspA on the surface of these bacteria.

### Example 9

This Example illustrates the close homology of portions of the pspA gene among different strains of pneumococci and the potential that such homology may be useful for molecular (DNA) approaches to detect pneumococci in tissues, body fluids and/or secretions and to identify pneumococci grown from patient samples.

Three DNA probes were employed, namely full length pspA, JY4323 (N-terminal HindIII to C-terminal KpnI) the N-terminal half of pspA, JY4306 (N-terminal HindIII to DraI at position 996 (see Figure 3) and most of the proline and repeat regions, JY4262 (BclI at position 1221 to BstNI at position 1832). Under stringency conditions requiring 95% identity, probes JY4323 and JY4262 reacted with cells of over 200 independent isolates of S.pneumoniae by Southern blot.

When the chromosomal DNA was cut with HindIII, there generally was observed that each of these probes detected two discrete bands whose exact size was dependent on the strain examined. In Rxl pneumococci, the two bands were 4.0 and 9.1 kb. The 4.0 kb band corresponded to pspA and was altered or absent in pspA mutants. The other band shares some homology with the coding regions for both the N-terminal and C-terminal halves of PspA but is not affected by pspA mutations. The JY4323 and JY4262 probes failed to react with another gram positive bacterium, Streptococcus pyogenes, and a gram negative bacterium, Salmonella typhimurium. The N-terminal probe, JY4306, recognized about one-third of the strains of pneumococci tested.

These results indicate that a sequence included in the proline/repeat region is shared by all strains of pneumococci and apparently not by other bacterial species. Sequences in the N-terminal half of the molecule appear to be more variable.

### Example 10

This Example illustrates the detection and determination of the location of epitopes in the α-helical N-terminal region of PspA.

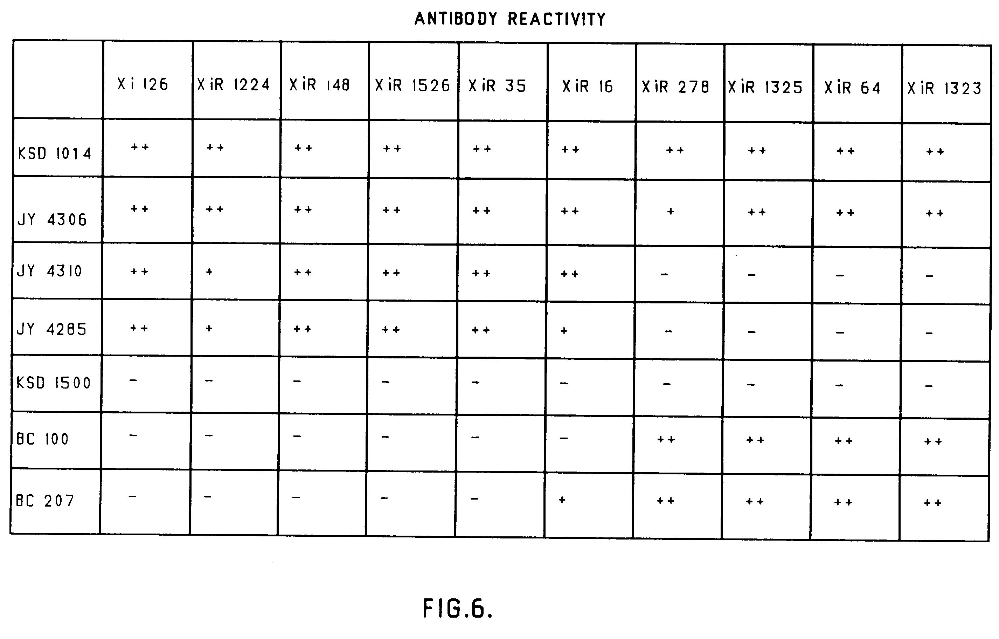

Monoclonal antibodies protective against pneumococcal infection in a mouse model, denoted by an asterisk in Figures 5, 6 and 7, were used to determine the location of epitopes for each antibody in the α-helical N-terminal region of PspA. The sites were mapped to fragments of PspA. The results are illustrated in Figures 5 to 7, with Figure 5 showing the deduced amino acid sequence for the N-terminal region of PspA and the general location of epitopes recognized by monoclonal antibodies, Figure 6 showing antibody reactivity with PspA fragments produced by various pspA gene segments, and Figure 7 showing the mapped location of epitopes in the PspA fragments produced by the different pspA gene segments.

Numbers 138, 193 and 261 in Figure 5 indicate break positions in the PspA fragments used to map the location of epitopes detected by monoclonal antibodies Xi1526, Xi126, XiR35, XiR38, XiR1224, XiR16, Xi64, XiR278, Xi1325 and Xi1323. The asterisk (*) after some of the antibodies denotes those which are able to protect against fatal pneumococcal infection with strain WU2 pneumococci.

In addition, the vertical lines to the right of the Figure indicate those areas predicted to have coiled-coil α-helical structure. The divisions to the left of the Figure indicate the mapped location of the epitopes for each antibody.

### SUMMARY OF DISCLOSURE

In summary of this disclosure, the present invention relates to a truncated PspA molecule capable of eliciting an immunoprotective response and hence containing the protective epitopes of PspA protein. Modifications are possible within the scope of this invention.

## Claims

1. A purified immunoprotective pneumococcal surface protein (PspA), comprising a truncated form of PspA which contains at least one immunoprotective epitope of the protein and up to 90% of the whole PspA protein and from which the functional cell membrane anchor region is absent.

2. The protein claimed in Claim 1, comprising approximately 50% of the whole PspA protein from which the cell membrane anchor region, the repeat region and the proline region are absent and comprising the 43 kD (apparent molecular size) N-terminal region of an 84 kD PspA protein.

3. The protein claimed in Claim 1 or Claim 2, comprising at least the N-terminal, protective epitope-containing region of the PspA protein.

4. The protein claimed in any one of Claims 1 to 3, comprising at least the N-terminal, α-helical protective epitope-containing region of the PspA protein and having a seven residue periodicity.

5. A biologically-pure, recombinant DNA molecule coding for a truncated form of PspA which contains up to 90% of the whole PspA protein and from which the functional cell membrane anchor region is absent, said DNA molecule having a coding sequence included in that set forth below:-

AAGCTTATGA TATAGAAATT TGTAACAAAA ATGTAATATA AAACACTTGA CAAATATTTA 60

CGGAGGAGGC TTATACTTAA TATAAGTATA GTCTGAAAAT GACTATCAGA AAAGAGGTAA 120

ATTTAGATGA ATAAGAAAAA AATGATTTTA ACAAGTCTAG CCAGCGTCGC TATCTTAGGG 180

GCTGGTTTTG TTGCGTCTCA GCCTACTGTT GTAAGAGCAG AAGAATCTCC CGTAGCCAGT 240

CAGTCTAAAG CTGAGAAAGA CTATGATGCA GCGAAGAAAG ATGCTAAGAA TGCGAAAAAA 300

GCAGTAGAAG ATGCTCAAAA GGCTTTAGAT GATGCAAAAG CTGCTCAGAA AAAATATGAC 360

GAGGATCAGA AGAAAACTGA GGAGAAAGCC GCGCTAGAAA AAGCAGCGTC TGAAGAGATG 420

GATAAGGCAG TGGCAGCAGT TCAACAAGCG TATCTAGCCT ATCAACAAGC TACAGACAAA 480

GCCGCAAAAG ACGCAGCAGA TAAGATGATA GATGAAGCTA AGAAACGCGA AGAAGAGGCA 540

AAAACTAAAT TTAATACTGT TCGAGCAATG GTAGTTCCTG AGCCAGAGCA GTTGGCTGAG 600

ACTAAGAAAA AATCAGAAGA AGCTAAACAA AAAGCACCAG AACTTACTAA AAAACTAGAA 660

GAAGCTAAAG CAAAATTAGA AGAGGCTGAG AAAAAAGCTA CTGAAGCCAA ACAAAAAGTG 720

GATGCTGAAG AAGTCGCTCC TCAAGCTAAA ATCGCTGAAT TGGAAAATCA AGTTCATAGA 780

CTAGAACAAG AGCTCAAAGA GATTGATGAG TCTGAATCAG AAGATTATGC TAAAGAAGGT 840

TTCCGTGCTC CTCTTCAATC TAAATTGGAT GCCAAAAAAG CTAAACTATC AAAACTTGAA 900

GAGTTAAGTG ATAAGATTGA TGAGTTAGAC GCTGAAATTG CAAAACTTGA AGATCAACTT 960

AAAGCTGCTG AAGAAAACAA TAATGTAGAA GACTACTTTA AAGAAGGTTT AGAGAAAACT 1020

ATTGCTGCTA AAAAAGCTGA ATTAGAAAAA ACTGAAGCTG ACCTTAAGAA AGCAGTTAAT 1080

GAGCCAGAAA AACCAGCTCC AGCTCCAGAA ACTCCAGCCC CAGAAGCACC AGCTGAACAA 1140

CCAAAACCAG CGCCGGCTCC TCAACCAGCT CCCGCACCAA AACCAGAGAA GCCAGCTGAA 1200

CAACCAAAAC CAGAAAAAAC AGATGATCAA CAAGCTGAAG AAGACTATGC TCGTAGATCA 1260

GAAGAAGAAT ATAATCGCTT GACTCAACAG CAACCGCCAA AAGCTGAAAA ACCAGCTCCT 1320

GCACCAAAAA CAGGCTGGAA ACAAGAAAAC GGTATGTGGT ACTTCTACAA TACTGATGGT 1380

TCAATGGCGA CAGGATGGCT CCAAAACAAC GGTTCATGGT ACTACCTCAA CAGCAATGGT 1440

GCTATGGCTA CAGGTTGGCT CCAATACAAT GGTTCATGGT ATTACCTCAA CGCTAACGGC 1500

GCTATGGCAA CAGGTTGGGC TAAAGTCAAC GGTTCATGGT ACTACCTCAA CGCTAATGGT 1560

GCTATGGCTA CAGGTTGGCT CCAATACAAC GGTTCATGGT ATTACCTCAA CGCTAACGGC 1620

GCTATGGCAA CAGGTTGGGC TAAAGTCAAC GGTTCATGGT ACTACCTCAA CGCTAATGGT 1680

GCTATGGCTA CAGGTTGGCT CCAATACAAC GGTTCATGGT ACTACCTCAA CGCTAACGGT 1740

GCTATGGCTA CAGGTTGGGC TAAAGTCAAC GGTTCATGGT ACTACCTCAA CGCTAATGGT 1800

GCTATGGCAA CAGGTTGGGT GAAAGATGGA GATACCTGGT ACTATCTTGA AGCATCAGGT 1860

GCTATGAAAG CAAGCCAATG GTTCAAAGTA TCAGATAAAT GGTACTATGT CAATGGTTTA 1920

GGTGCCCTTG CAGTCAACAC AACTGTAGAT GGCTATAAAG TCAATGCCAA TGGTGAATGG 1980

GTTTAAGCCG ATTAAATTAA AGCATGTTAA GAACATTTGA CATTTTAATT TTGAAACAAA 2040

GATAAGGTTC GATTGAATAG ATTTATGTTC GTATTCTTTA GGTAC 2085

6. A DNA molecule claimed in Claim 5, coding for at least part of an α-helical coiled coil region of the PspA protein, at least part of a proline rich region of the PspA protein, or at least part of a repeat region of the PspA protein.

7. A DNA molecule claimed in Claim 5 or Claim 6, in the form of a DNA probe for the detection of pneumococcal DNA in a sample characterised by a conserved sequence of the pspA gene, said sequence selected from the group consisting of a nucleic acid sequence encoding a proline-rich region of PspA, a nucleic acid sequence encoding a repeats region of PspA and a nucleic acid sequence encoding a proline-rich region and a repeats region of PspA.

8. A DNA molecule claimed in Claims 5 or 6, in the form of a DNA primer for effecting polymerase chain reaction of pneumococcal DNA, characterised by a conserved sequence of the pspA gene.

9. A DNA molecule claimed in Claim 8, in which said conserved sequence is located in the proline-rich region of the gene, the repeat region in the gene or both.

10. The DNA molecule claimed in Claim 7, which is DNA probe JY4262, consisting of bases 1221 to 1832 of the sequence shown in Claim 5.

11. The DNA molecule claimed in Claim 8, in the form of a DNA primer, in which said primer has the nucleotide sequence: 5'-CCGGATCCAGCTCCTGCACCAAAAC-3' (LSM1) or

5'-GCGCTGCGACGGCTTAAACCCATTCACCATTGG-3' (LSM2)

12. A DNA molecule claimed in Claim 5, which codes for a truncated form of the PspA protein which contains approximately 50% of the whole PspA protein from which the cell membrane anchor region, the repeats region and the proline region are absent.

13. A DNA molecule claimed in Claim 5 or Claim 12, which is linked by a translationally in-frame genetic fusion to a gene coding for another protein.

14. A DNA molecule claimed in Claim 13, in which said gene coding for another protein is a ctxB gene.

15. A method for forming an immunoprotective truncated PspA protein, characterised by:
(a) effecting insertion-duplication mutagenesis of a bacterium with a pspA gene resulting in the coding of a truncated expressible pneumococcal surface protein (PspA) from which the functional cell membrane anchor region is absent, or
(b) fusing a pspA gene coding for a truncated form of PspA protein from which the functional cell membrane anchor region is absent with a gene coding for another protein to form a fusion protein clone and transforming a bacterium with said fusion protein clone,
growing said bacterium to effect expression of a truncated PspA protein or a fusion protein comprising truncated PspA and said other protein, and
isolating the expressed protein.

16. A method as claimed in Claim 15, in which said bacterium is an S.pneumoniae strain, an E.coli strain or a Mycobacteria strain.

17. A method as claimed in Claims 15 or 16, in which said bacterium is S.pneumoniae strain JY2008 (ATCC 55143), or an S.pneumoniae strain producing a truncated PspA protein corresponding to an N-terminal portion of PspA protein of 21 kDa molecular weight, or a truncated PspA protein corresponding to an N-terminal portion of PspA protein of 18 kDa molecular weight, or strain JY4306 (ATCC 68522), or BCG.

18. A method as claimed in any one of Claims 15 or 16, in which said gene coding for another protein is the ctxB gene coding for the B-subunit of cholera toxin (CTB).

19. A method as claimed in Claim 18, in which said fusion protein comprises an acid labile sequence joining said truncated form of PspA protein and CTB and said PspA protein and CTB are separated from each other by dilute acid treatment.

20. A method as claimed in any one of Claims 15 to 17, in which said fusion protein is isolated from culture medium by adsorption to a G_{M1} affinity column, from which said fusion protein is eluted.

21. A mutated strain of Streptococcus pneumoniae, Eschericia coli or Mycobacterium characterised by a pspA gene coding for a truncated expressible pneumococcal surface protein (PspA) from which the cell membrane anchor region is absent.

22. A strain as claimed in Claim 21, which is the JY2008 (ATCC 55143) strain of S.pneumoniae, or an S.pneumoniae strain producing a truncated PspA protein corresponding to an N-terminal portion of PspA protein of 21 kDa molecular weight, or a truncated PspA protein corresponding to an N-terminal portion of PspA of 18 kDa molecular weight, or the strain JY4306 (ATCC 68522), or a mutated BCG.

23. A strain as claimed in Claims 21 or 22, in which the pspA gene coding for a truncated PspA protein is linked to a gene coding for another protein.

24. A strain as claimed in Claim 23, in which said gene coding for another protein is the ctxB gene coding for the B-subunit for cholera toxin.

25. A vaccine against pneumococcal infection, characterised by, as an immunogenically-active component, the protein defined in any one of Claims 1 to 4, or the expression product of a DNA molecule defined in any one of Claims 5, 6, 12, 13 or 14, or a live-attenuated or killed gram positive bacteria containing a gene coding for the protein defined in any one of Claims 1 to 4 or a DNA molecule defined in any one of Claims 5, 6, 12, 13 or 14 or the protein produced by the method of any one of Claims 15 to 20.

26. A vaccine as claimed in Claim 25, in which said protein is conjugated to a normally weakly-immunogenic or non-immunogenic protection-eliciting molecule.

27. A vaccine claimed in Claim 25, in which said gram positive bacteria is Streptococcus pneumoniae or Mycobacterium tuberculosis.

28. A solid phase immunoabsorbent assay, characterised by a coating of a truncated form of PspA protein as claimed in any one of Claims 1 to 4 on a solid substrate wherein said PspA protein is fused to the B-subunit of cholera toxin (CTB) which is bound to monosinloganglioside (G_{M1}) coating said solid substrate.

## Patentansprüche

1. Gereinigtes immunprotektives Pneumokokken-Oberflächen-protein (PspA), umfassend eine verkürzte Form von PspA, die mindestens ein immunprotektives Epitop des Proteins und bis zu 90 % des Gesamt-PspA-Proteins enthält und der der funktionelle Zellmembranankerbereich fehlt.

2. Protein nach Anspruch 1, umfassend etwa 50 % des Gesamt-PspA-Proteins, dem der Zellmembranankerbereich, der Wiederholungsbereich und der Prolinbereich fehlen und das den N-terminalen Bereich von 43 kD (apparentes Molekulargewicht) eines PspA-Proteins von 84 kD umfaßt.

3. Protein nach Anspruch 1 oder 2, das mindestens den N-terminalen, protektiven Epitop-enthaltenden Bereich des PspA-Proteins umfaßt.

4. Protein nach einem der Ansprüche 1 bis 3, das mindestens den N-terminalen, α-helikalen, protektiven Epitop-enthaltenden Bereich des PspA-Proteins umfaßt und eine Periodizität von 7 Resten aufweist.

5. Biologisch reines rekombinantes DNA-Molekül, das eine verkürzte Form von PspA codiert, die bis zu 90 % des Gesamt-PspA-Proteins enthält und der der funktionelle Zellmembranankerbereich fehlt, wobei das DNA-Molekül eine in der nachstehend dargelegten Sequenz eingeschlossene codierende Sequenz aufweist:

AAGCTTATGA TATAGAAATT TGTAACAAAA ATGTAATATA AAACACTTGA CAAATATTTA 60

CGGAGGAGGC TTATACTTAA TATAAGTATA GTCTGAAAAT GACTATCAGA AAAGAGGTAA 120

ATTTAGATGA ATAAGAAAAA AATGATTTTA ACAAGTCTAG CCAGCGTCGC TATCTTAGGG 180

GCTGGTTTTG TTGCGTCTCA GCCTACTGTT GTAAGAGCAG AAGAATCTCC CGTAGCCAGT 240

CAGTCTAAAG CTGAGAAAGA CTATGATGCA GCGAAGAAAG ATGCTAAGAA TGCGAAAAAA 300

GCAGTAGAAG ATGCTCAAAA GGCTTTAGAT GATGCAAAAG CTGCTCAGAA AAAATATGAC 360

GAGGATCAGA AGAAAACTGA GGAGAAAGCC GCGCTAGAAA AAGCAGCGTC TGAAGAGATG 420

GATAAGGCAG TGGCAGCAGT TCAACAAGCG TATCTAGCCT ATCAACAAGC TACAGACAAA 480

GCCGCAAAAG ACGCAGCAGA TAAGATGATA GATGAAGCTA AGAAACGCGA AGAAGAGGCA 540

AAAACTAAAT TTAATACTGT TCGAGCAATG GTAGTTCCTG AGCCAGAGCA GTTGGCTGAG 600

ACTAAGAAAA AATCAGAAGA AGCTAAACAA AAAGCACCAG AACTTACTAA AAAACTAGAA 660

GAAGCTAAAG CAAAATTAGA AGAGGCTGAG AAAAAAGCTA CTGAAGCCAA ACAAAAAGTG 720

GATGCTGAAG AAGTCGCTCC TCAAGCTAAA ATCGCTGAAT TGGAAAATCA AGTTCATAGA 780

CTAGAACAAG AGCTCAAAGA GATTGATGAG TCTGAATCAG AAGATTATGC TAAAGAAGGT 840

TTCCGTGCTC CTCTTCAATC TAAATTGGAT GCCAAAAAAG CTAAACTATC AAAACTTGAA 900

GAGTTAAGTG ATAAGATTGA TGAGTTAGAC GCTGAAATTG CAAAACTTGA AGATCAACTT 960

AAAGCTGCTG AAGAAAACAA TAATGTAGAA GACTACTTTA AAGAAGGTTT AGAGAAAACT 1020

ATTGCTGCTA AAAAAGCTGA ATTAGAAAAA ACTGAAGCTG ACCTTAAGAA AGCAGTTAAT 1080

GAGCCAGAAA AACCAGCTCC AGCTCCAGAA ACTCCAGCCC CAGAAGCACC AGCTGAACAA 1140

CCAAAACCAG CGCCGGCTCC TCAACCAGCT CCCGCACCAA AACCAGAGAA GCCAGCTGAA 1200

CAACCAAAAC CAGAAAAAAC AGATGATCAA CAAGCTGAAG AAGACTATGC TCGTAGATCA 1260

GAAGAAGAAT ATAATCGCTT GACTCAACAG CAACCGCCAA AAGCTGAAAA ACCAGCTCCT 1320

GCACCAAAAA CAGGCTGGAA ACAAGAAAAC GGTATGTGGT ACTTCTACAA TACTGATGGT 1380

TCAATGGCGA CAGGATGGCT CCAAAACAAC GGTTCATGGT ACTACCTCAA CAGCAATGGT 1440

GCTATGGCTA CAGGTTGGCT CCAATACAAT GGTTCATGGT ATTACCTCAA CGCTAACGGC 1500

GCTATGGCAA CAGGTTGGGC TAAAGTCAAC GGTTCATGGT ACTACCTCAA CGCTAATGGT 1560

GCTATGGCTA CAGGTTGGCT CCAATACAAC GGTTCATGGT ATTACCTCAA CGCTAACGGC 1620

GCTATGGCAA CAGGTTGGGC TAAAGTCAAC GGTTCATGGT ACTACCTCAA CGCTAATGGT 1680

GCTATGGCTA CAGGTTGGCT CCAATACAAC GGTTCATGGT ACTACCTCAA CGCTAACGGT 1740

GCTATGGCTA CAGGTTGGGC TAAAGTCAAC GGTTCATGGT ACTACCTCAA CGCTAATGGT 1800

GCTATGGCAA CAGGTTGGGT GAAAGATGGA GATACCTGGT ACTATCTTGA AGCATCAGGT 1860

GCTATGAAAG CAAGCCAATG GTTCAAAGTA TCAGATAAAT GGTACTATGT CAATGGTTTA 1920

GGTGCCCTTG CAGTCAACAC AACTGTAGAT GGCTATAAAG TCAATGCCAA TGGTGAATGG 1980

GTTTAAGCCG ATTAAATTAA AGCATGTTAA GAACATTTGA CATTTTAATT TTGAAACAAA 2040

GATAAGGTTC GATTGAATAG ATTTATGTTC GTATTCTTTA GGTAC 2085

6. DNA-Molekül nach Anspruch 5, das mindestens einen Teil eines α-helikalen, in sich verknäulten Knäuel-Bereichs des PspA-Proteins, mindestens einen Teil eines prolinreichen Bereichs des PspA-Proteins oder mindestens einen Teil eines Wiederholungsbereichs des PspA-Proteins codiert.

7. DNA-Molekül nach Anspruch 5 oder 6 in Form einer DNA-Sonde zum Nachweis von Pneumokokken-DNA in einer Probe, das durch eine konservierte Sequenz des PspA-Gens gekennzeichnet ist, die eine einen prolinreichen Bereich von PspA codierende Nucleinsäure, eine einen Wiederholungsbereich von PspA codierende Nucleinsäure oder eine einen prolinreichen Bereich und einen Wiederholungsbereich von PspA codierende Nucleinsäure ist.

8. DNA-Molekül nach Anspruch 5 oder 6 in Form eines DNA-Primers zur Durchführung einer Polymerasekettenreaktion bei Pneumokokken-DNA, das durch eine konservierte Sequenz des PspA-Gens gekennzeichnet ist.

9. DNA-Molekül nach Anspruch 8, in dem die konservierte Sequenz in dem prolinreichen Bereich des Gens, in dem Wiederholungsbereich des Gens oder in beiden liegt.

10. DNA-Molekül nach Anspruch 7, das die aus den Basen 1221 bis 1832 der in Anspruch 5 gezeigten Sequenz bestehende DNA-Sonde JY4262 ist.

11. DNA-Molekül nach Anspruch 8 in Form eines DNA-Primers, wobei der Primer die folgende Nucleotidsequenz aufweist: 5'-CCGGATCCAGCTCCTGCACCAAAAC-3'          (LSM1) oder

5'-GCGCTGCGACGGCTTAAACCCATTCACCATTGG-3' (LSM2)

12. DNA-Molekül nach Anspruch 5, das eine verkürzte Form des PspA-Proteins codiert, die etwa 50 % des Gesamt-PspA-Proteins enthält und der der Zellmembranankerbereich, der Wiederholungsbereich und der Prolinbereich fehlen.

13. DNA-Molekül nach Anspruch 5 oder 12, das mit einem ein anderes Protein codierenden Gen durch eine die Translation im Leserahmen ermöglichende genetische Fusion verknüpft ist.

14. DNA-Molekül nach Anspruch 13, in dem das ein anderes Protein codierende Gen ein ctxB-Gen ist.

15. Verfahren zur Erzeugung eines immunprotektiven verkürzten PspA-Proteins, gekennzeichnet durch:
(a) Herbeiführung einer Insertion-Duplikationsmutagenese eines Bakteriums mit einem pspA-Gen, was zu der Codierung eines verkürzten exprimierbaren Pneumokokkenoberflächenproteins (PspA) führt, dem der funktionelle Zellmembranankerbereich fehlt, oder
(b) Fusion eines pspA-Gens, das eine verkürzte Form eines PspA-Proteins codiert, dem der funktionelle Zellmembranankerbereich fehlt, mit einem ein anderes Protein codierenden Gen zur Erzeugung eines Fusionsproteinclons und Transformation eines Bakteriums mit dem Fusionsproteinclon,
Züchtung des Bakteriums zur Herbeiführung der Expression eines verkürzten PspA-Proteins oder eines das verkürzte PspA und das andere Protein enthaltenden Fusionsproteins und
Isolierung des exprimierten Proteins.

16. Verfahren nach Anspruch 15, wobei das Bakterium ein S. pneumoniae-Stamm, ein E. coli-Stamm oder ein Mycobacterium-Stamm ist.

17. Verfahren nach Anspruch 15 oder 16, wobei das Bakterium der S. pneumoniae-Stamm JY2008 (ATCC 55143) ist oder ein S. pneumoniae-Stamm, der ein verkürztes PspA-Protein, das einem N-terminalen Teil eines PspA-Proteins mit einem Molekulargewicht von 21 kDa entspricht, oder ein verkürztes PspA-Protein, das einem N-terminalen Teil eines PspA-Proteins mit einem Molekulargewicht von 18 kDa entspricht, produziert, oder Stamm JY4306 (ATCC 68522) oder BCG.

18. Verfahren nach Anspruch 15 oder 16, wobei das ein anderes Protein codierende Gen das ctxB-Gen ist, das die B-Untereinheit des Choleratoxins (CTB) codiert.

19. Verfahren nach Anspruch 18, wobei das Fusionsprotein eine säurelabile Sequenz enthält, die die verkürzte Form des PspA-Proteins und CTB verbindet, und wobei das PspA-Protein und CTB durch eine Behandlung mit verdünnter Säure voneinander getrennt werden.

20. Verfahren nach einem der Ansprüche 15 bis 17, wobei das Fusionsprotein aus dem Kulturmedium durch Adsorption an eine G_{M1}-Affinitätssäule isoliert wird, von der das Fusionsprotein eluiert wird.

21. Mutierter Streptococcus pneumoniae-, Escherichia coli- oder Mycobacterium-Stamm, der durch ein pspA-Gen gekennzeichnet ist, das ein verkürztes exprimierbares Pneumokokken-Oberflächenprotein (PspA) codiert, dem der Zellmembranankerbereich fehlt.

22. Stamm nach Anspruch 21, der der JY2008 (ATCC 55143)-Stamm von S. pneumoniae ist, oder ein S.pneumoniae-Stamm, der ein verkürztes PspA-Protein, das einem N-terminalen Bereich des PspA-Proteins mit einem Molekulargewicht von 21 kDa entspricht, oder ein verkürztes PspA-Protein, das einem N-terminalen Bereich des PspA-Proteins mit einem Molekulargewicht von 18 kDa entspricht, produziert, oder der Stamm JY4306 (ATCC 68522) oder ein mutierter BCG.

23. Stamm nach Anspruch 21 oder 22, wobei das ein verkürztes PspA-Protein codierende pspA-Gen mit einem ein anderes Protein codierenden Gen verknüpft ist.

24. Stamm nach Anspruch 23, wobei das ein anderes Protein codierende Gen das ctxB-Gen ist, das die B-Untereinheit des Choleratoxins codiert.

25. Impfstoff gegen eine Pneumokokken-Infektion, gekennzeichnet durch das Protein gemäß der Definition in einem der Ansprüche 1 bis 4, das Expressionsprodukt eines DNA-Moleküls gemäß der Definition in einem der Ansprüche 5, 6, 12, 13 oder 14, ein lebendes-attenuiertes oder abgetötetes gram-positives Bakterium, das ein das Protein gemäß der Definition in einem der Ansprüche 1 bis 4 codierendes Gen oder ein DNA-Molekül gemäß der Definition in einem der Ansprüche 5, 6, 12, 13 oder 14 enthält, oder das Protein, das durch das Verfahren nach einem der Ansprüche 15 bis 20 produziert wird, als immunologisch aktiver Bestandteil.

26. Impfstoff nach Anspruch 25, wobei das Protein mit einem normalerweise schwach immunogenen oder nicht-immunogenen, Schutz hervorrufenden Molekül konjugiert ist.

27. Impfstoff nach Anspruch 25, wobei das gram-positive Bakterium Streptococcus pneumoniae oder Mycobacterium tuberculosis ist.

28. Festphasen-Immunadsorptions-Test, gekennzeichnet durch die Beschichtung eines Festsubstrates mit einer verkürzten Form des PspA-Proteins nach einem der Ansprüche 1 bis 4, wobei das PspA-Protein mit der B-Untereinheit des Choleratoxins (CTB) fusioniert ist, die an das Festsubstrat beschichtendes Monosialogangliosid (G_{M1}) gebunden ist.

## Revendications

1. Protéine de surface pneumococcique immunoprotectrice purifiée (PspA), comprenant une forme tronquée de PspA qui contient au moins un épitope immunoprotecteur de la protéine et jusqu'à 90% de la protéine PspA totale et dont la région fonctionnelle d'ancrage dans la membrane cellulaire est absente.

2. Protéine selon la revendication 1, comprenant approximativement 50% de la protéine PspA totale dont la région d'ancrage dans la membrane cellulaire, la région de répétitions et la région de la proline sont absentes et comprenant la région N-terminale de 43 kD (taille moléculaire apparente) d'une protéine PspA de 84 kD.

3. Protéine selon la revendication 1 ou 2, comprenant au moins la région N-terminale contenant un épitope protecteur de la protéine PspA.

4. Protéine selon l'une quelconque des revendications 1 à 3, comprenant au moins la région N-terminale contenant un épitope protecteur en hélice a de la protéine PspA et possédant une périodicité de sept résidus.

5. Molécule d'ADN recombinant biologiquement pure codant pour une forme tronquée de PspA qui contient jusqu'à 90% de la protéine PspA totale et dont la région fonctionnelle d'ancrage dans la membrane cellulaire est absente, ladite molécule d'ADN possédant une séquence codante comprise dans celle présentée ci-dessous:

AAGCTTATGA TATAGAAATT TGTAACAAAA ATGTAATATA AAACACTTGA CAAATATTTA 60

CGGAGGAGGC TTATACTTAA TATAAGTATA GTCTGAAAAT GACTATCAGA AAAGAGGTAA 120

ATTTAGATGA ATAAGAAAAA AATGATTTTA ACAAGTCTAG CCAGCGTCGC TATCTTAGGG 180

GCTGGTTTTG TTGCGTCTCA GCCTACTGTT GTAAGAGCAG AAGAATCTCC CGTAGCCAGT 240

CAGTCTAAAG CTGAGAAAGA CTATGATGCA GCGAAGAAAG ATGCTAAGAA TGCGAAAAAA 300

GCAGTAGAAG ATGCTCAAAA GGCTTTAGAT GATGCAAAAG CTGCTCAGAA AAAATATGAC 360

GAGGATCAGA AGAAAACTGA GGAGAAAGCC GCGCTAGAAA AAGCAGCGTC TGAAGAGATG 420

GATAAGGCAG TGGCAGCAGT TCAACAAGCG TATCTAGCCT ATCAACAAGC TACAGACAAA 480

GCCGCAAAAG ACGCAGCAGA TAAGATGATA GATGAAGCTA AGAAACGCGA AGAAGAGGCA 540

AAAACTAAAT TTAATACTGT TCGAGCAATG GTAGTTCCTG AGCCAGAGCA GTTGGCTGAG 600

ACTAAGAAAA AATCAGAAGA AGCTAAACAA AAAGCACCAG AACTTACTAA AAAACTAGAA 660

GAAGCTAAAG CAAAATTAGA AGAGGCTGAG AAAAAAGCTA CTGAAGCCAA ACAAAAAGTG 720

GATGCTGAAG AAGTCGCTCC TCAAGCTAAA ATCGCTGAAT TGGAAAATCA AGTTCATAGA 780

CTAGAACAAG AGCTCAAAGA GATTGATGAG TCTGAATCAG AAGATTATGC TAAAGAAGGT 840

TTCCGTGCTC CTCTTCAATC TAAATTGGAT GCCAAAAAAG CTAAACTATC AAAACTTGAA 900

GAGTTAAGTG ATAAGATTGA TGAGTTAGAC GCTGAAATTG CAAAACTTGA AGATCAACTT 960

AAAGCTGCTG AAGAAAACAA TAATGTAGAA GACTACTTTA AAGAAGGTTT AGAGAAAACT 1020

ATTGCTGCTA AAAAAGCTGA ATTAGAAAAA ACTGAAGCTG ACCTTAAGAA AGCAGTTAAT 1080

GAGCCAGAAA AACCAGCTCC AGCTCCAGAA ACTCCAGCCC CAGAAGCACC AGCTGAACAA 1140

CCAAAACCAG CGCCGGCTCC TCAACCAGCT CCCGCACCAA AACCAGAGAA GCCAGCTGAA 1200

CAACCAAAAC CAGAAAAAAC AGATGATCAA CAAGCTGAAG AAGACTATGC TCGTAGATCA 1260

GAAGAAGAAT ATAATCGCTT GACTCAACAG CAACCGCCAA AAGCTGAAAA ACCAGCTCCT 1320

GCACCAAAAA CAGGCTGGAA ACAAGAAAAC GGTATGTGGT ACTTCTACAA TACTGATGGT 1380

TCAATGGCGA CAGGATGGCT CCAAAACAAC GGTTCATGGT ACTACCTCAA CAGCAATGGT 1440

GCTATGGCTA CAGGTTGGCT CCAATACAAT GGTTCATGGT ATTACCTCAA CGCTAACGGC 1500

GCTATGGCAA CAGGTTGGGC TAAAGTCAAC GGTTCATGGT ACTACCTCAA CGCTAATGGT 1560

GCTATGGCTA CAGGTTGGCT CCAATACAAC GGTTCATGGT ATTACCTCAA CGCTAACGGC 1620

GCTATGGCAA CAGGTTGGGC TAAAGTCAAC GGTTCATGGT ACTACCTCAA CGCTAATGGT 1680

GCTATGGCTA CAGGTTGGCT CCAATACAAC GGTTCATGGT ACTACCTCAA CGCTAACGGT 1740

GCTATGGCTA CAGGTTGGGC TAAAGTCAAC GGTTCATGGT ACTACCTCAA CGCTAATGGT 1800

GCTATGGCAA CAGGTTGGGT GAAAGATGGA GATACCTGGT ACTATCTTGA AGCATCAGGT 1860

GCTATGAAAG CAAGCCAATG GTTCAAAGTA TCAGATAAAT GGTACTATGT CAATGGTTTA 1920

GGTGCCCTTG CAGTCAACAC AACTGTAGAT GGCTATAAAG TCAATGCCAA TGGTGAATGG 1980

GTTTAAGCCG ATTAAATTAA AGCATGTTAA GAACATTTGA CATTTTAATT TTGAAACAAA 2040

GATAAGGTTC GATTGAATAG ATTTATGTTC GTATTCTTTA GGTAC 2085

6. Molécule d'ADN selon la revendication 5, codant pour au moins une partie d'une région bispiralée en hélice α de la protéine PspA, pour au moins une partie d'une région riche en proline de la protéine PspA, ou pour au moins une partie d'une région de répétitions de la protéine PspA.

7. Molécule d'ADN selon la revendication 5 ou 6, sous forme d'une sonde d'ADN servant à la détection d'ADN pneumococcique dans un échantillon, caractérisée par une séquence conservée du gène pspA, ladite séquence étant choisie dans le groupe constitué par une séquence d'acides nucléiques codant pour une région riche en proline de PspA, une séquence d'acides nucléiques codant pour une région de répétitions de PspA et une séquence d'acides nucléiques codant pour une région riche en proline et une région de répétitions de PspA.

8. Molécule d'ADN selon la revendication 5 ou 6, sous forme d'une amorce d'ADN pour effectuer l'amplification en chaîne par polymérase d'ADN pneumococcique, caractérisée par une séquence conservée du gène pspA.

9. Molécule d'ADN selon la revendication 8, dans laquelle ladite séquence conservée est située dans la région riche en proline du gène, dans la région de répétitions du gène ou dans les deux à la fois.

10. Molécule d'ADN selon la revendication 7, qui est une sonde d'ADN JY4262, constituée des bases 1221 à 1832 de la séquence présentée dans la revendication 5.

11. Molécule d'ADN selon la revendication 8, sous forme d'une amorce d'ADN, dans laquelle ladite amorce possède la séquence nucléotidique: 5'-CCGGATCCAGCTCCTGCACCAAAAC-3' (LSM1) ou

5'-GCGCTGCGACGGCTTAAACCCATTCACCATTGG-3'(LSM2)

12. Molécule d'ADN selon la revendication 5, qui code pour une forme tronquée de la protéine PspA, qui contient approximativement 50% de la protéine PspA totale dont la région d'ancrage dans la membrane cellulaire, la région de répétitions et la région de la proline sont absentes.

13. Molécule d'ADN selon la revendication 5 ou la revendication 12, qui est reliée par fusion génétique traductionnelle, respectant les cadres de lecture, à un gène codant pour une autre protéine.

14. Molécule d'ADN selon la revendication 13, dans laquelle ledit gène codant pour une autre protéine est une gène ctxB.

15. Procédé pour former une protéine PspA tronquée immunoprotectrice, caractérisé par les étapes consistant à:
(a) effectuer une mutagénèse par insertion-duplication d'une bactérie avec un gène pspA, ce qui conduit au codage d'une protéine de surface pneumococcique tronquée expressible (PspA) dont la région fonctionnelle d'ancrage dans la membrane cellulaire est absente, ou
(b) fusionner un gène pspA codant pour une forme tronquée de la protéine PspA dont la région fonctionnelle d'ancrage dans la membrane cellulaire est absente, avec un gène codant pour une autre protéine pour former un clone de protéine de fusion, et transformer une bactérie avec ledit clone de protéine de fusion,
faire pousser ladite bactérie pour obtenir l'expression d'une protéine PspA tronquée ou d'une protéine de fusion comprenant la PspA tronquée et ladite autre protéine, et
isoler la protéine exprimée.

16. Procédé selon la revendication 15, dans lequel ladite bactérie est une souche de S. pneumoniae, une souche de E. coli ou une souche de Mycobacteria.

17. Procédé selon la revendication 15 ou 16, dans lequel ladite bactérie est la souche JY2008 de S. pneumoniae (ATCC 55143), ou une souche de S. pneumoniae produisant une protéine PspA tronquée qui correspond à une portion N-terminale de la protéine PspA ayant un poids moléculaire de 21 kDa, ou une protéine PspA tronquée qui correspond à une portion N-terminale de la protéine PspA ayant un poids moléculaire de 18 kDa, ou la souche JY4306 (ATCC 68522), ou BCG.

18. Procédé selon l'une quelconque des revendications 15 et 16, dans lequel ledit gène codant pour une autre protéine est le gène ctxB qui code pour la sous-unité B de la toxine cholérique (CTB).

19. Procédé selon la revendication 18, dans lequel ladite protéine de fusion comprend une séquence acido-labile reliant ladite forme tronquée de la protéine PspA et CTB, ladite protéine PspA et CTB étant séparées l'une de l'autre par traitement à l'acide dilué.

20. Procédé selon l'une quelconque des revendications 15 à 17, dans lequel ladite protéine de fusion est isolée du milieu de culture par adsorption sur une colonne d'affinité de G_{M1} à partir de laquelle ladite protéine de fusion est éluée.

21. Souche mutée de Streptococcus pneumoniae, de Escherichia coli ou de Mycobacterium, caractérisée par un gène pspA codant pour une protéine de surface pneumococcique tronquée expressible (PspA) dont la région d'ancrage dans la membrane cellulaire est absente.

22. Souche selon la revendication 21, qui est la souche JY2008 (ATCC 55143) de S. pneumoniae, ou une souche de S. pneumoniae produisant une protéine PspA tronquée qui correspond à une portion N-terminale de la protéine PspA ayant un poids moléculaire de 21 kDa, ou une protéine PspA tronquée qui correspond à une portion N-terminale de PspA ayant un poids moléculaire de 18 kDa, ou la souche JY4306 (ATCC 68522), ou une BCG mutée.

23. Souche selon la revendication 21 ou 22, dans laquelle le gène pspA codant pour une protéine PspA tronquée est relié à un gène codant pour une autre protéine.

24. Souche selon la revendication 23, dans laquelle ledit gène codant pour une autre protéine est le gène ctxB qui code pour la sous-unité B de la toxine cholérique.

25. Vaccin contre une infection à pneumocoques, caractérisé par la protéine définie dans l'une quelconque des revendications 1 à 4, ou le produit d'expression d'une molécule d'ADN définie dans l'une quelconque des revendications 5, 6, 12, 13 et 14, ou une bactérie Gram positif vivante atténuée ou tuée contenant un gène codant pour la protéine définie dans l'une quelconque des revendications 1 à 4 ou une molécule d'ADN définie dans l'une quelconque des revendications 5, 6, 12, 13 et 14 ou la protéine produite par le procédé selon l'une quelconque des revendications 15 à 20, à titre de composant actif sur le plan immunogène.

26. Vaccin selon la revendication 25, dans lequel ladite protéine est conjuguée à une molécule normalement faiblement immunogène ou à une molécule non immunogène déclenchant une protection.

27. Vaccin selon la revendication 25, dans lequel ladite bactérie Gram positif est Streptococcus pneumoniae ou Mycobacterium tuberculosis.

28. Technique de dosage par immunocaptation en phase solide, caractérisée par un revêtement d'une forme tronquée de protéine PspA selon l'une quelconque des revendications 1 à 4 sur un substrat solide, dans lequel ladite protéine PspA est fusionnée à la sous-unité B de la toxine cholérique (CTB) qui est fixée au monosinloganglioside (G_{M1}) recouvrant ledit substrat solide.
